(19) Europäisches Patentamt
European Patent Office
Office européen des brevets

(11) Veröffentlichungsnummer: **0 160 930**
**A2**

(12) # EUROPÄISCHE PATENTANMELDUNG

(21) Anmeldenummer: **85105316.5**

(22) Anmeldetag: **02.05.85**

(51) Int. Cl.⁴: **C 07 D 405/04**
**A 01 N 43/653, A 01 N 43/50**

(30) Priorität: **11.05.84 DE 3417467**

(43) Veröffentlichungstag der Anmeldung:
**13.11.85 Patentblatt 85/46**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Böckmann, Klaus, Dr.**
**Andreas-Gryphius-Strasse 7**
**D-5000 Köln 80(DE)**

(72) Erfinder: **Jäger, Gerhard, Dr.**
**Gellertstrasse 18**
**D-5090 Leverkusen 1(DE)**

(72) Erfinder: **Brandes, Wilhelm, Dr.**
**Eichendorffstrasse 3**
**D-5653 Leichlingen 1(DE)**

(72) Erfinder: **Reinecke, Paul, Dr.**
**Steinstrasse 8**
**D-5090 Leverkusen 3(DE)**

(54) **Cyclische Azolylvinylether.**

(57) Es werden neue cyclische Azolylvinylether der Formel

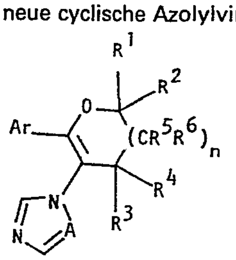

(I)

in welcher

A für ein Stickstoffatom oder die CH-Gruppe steht,
Ar für gegebenenfalls substituiertes Phenyl steht,
$R^1$ bis $R^6$ gleich oder verschieden sind und für Wasserstoff oder Alkyl stehen und
n für 0 oder 1 steht,

und deren Säureadditions-Salze und Metallsalz-Komplexe bereitgestellt.

Die Erfindung betrifft weiterhin ein Verfahren zur Herstellung der Verbindungen (I) sowie ihre Verwendung als Fungizide.

EP 0 160 930 A2

Croydon Printing Company Ltd

BAYER AKTIENGESELLSCHAFT 5090 Leverkusen, Bayerwerk

Konzernverwaltung RP
Patentabteilung Slr/Er/Ke

Ia

## Cyclische Azolylvinylether

Die vorliegende Erfindung betrifft neue cyclische Azolylvinylether, ein Verfahren zu ihrer Herstellung sowie ihre Verwendung als Fungizide.

Es ist bereits bekannt, daß bestimmte 1-Ethen-azolyl-Derivate, wie beispielsweise 2-(4-Chlorphenoxy)-4,4-dimethyl-1-(imidazol-1-yl)-1-penten-3-on, gute fungizide Eigenschaften aufweisen (vergleiche DE-OS 2846 980).
Außderdem ist bereits bekannt, daß bestimmte Arylvinylazolylether gute fungizide Eigenschaften aufweisen (vergleiche DE-OS 2757113, DE-OS 2839388 und EP 0079856).
Weiterhin ist bereits bekannt, daß Disulfide, wie beispielsweise Zinkethylen-1,2-bisdithiocarbamidat, gute Mittel zur Bekämpfung von pilzlichen Pflanzenkrankheiten sind (vergleiche R. Wegler, 'Chemie der Pflanzenschutz- und Schädlingsbekämpfungsmittel', Band 2, S.59ff, Springer Verlag 1970).

Die Wirkung dieser Verbindungen ist jedoch in bestimmten Indikationsbereichen, insbesondere bei niedrigen Aufwandmengen und -Konzentrationen, nicht immer voll befriedigend.

Es wurden neue cyclische Azolylvinylether der allgemeinen Formel (I)

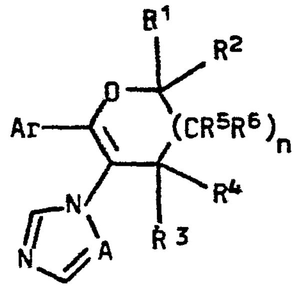

(I)

in welcher

A für ein Stickstoffatom oder die CH-Gruppe steht,
Ar für gegebenenfalls substituiertes Phenyl steht,
$R^1$ bis $R^6$ gleich oder verschieden sind und für Wasserstoff oder Alkyl stehen und
n für 0 oder 1 steht,

und deren Säureadditions-Salze und Metallsalz-Komplexe gegefunden.

Weiterhin wurde gefunden, daß man die neuen cyclischen Azolylvinylether der Formel (I) erhält, wenn man Azolylvinyl-Ketone der Formel (II)

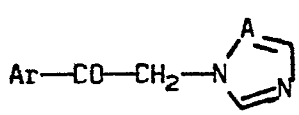

(II)

in welcher

A und Ar die oben angegebene Bedeutung haben,

mit Verbindungen der Formel (III)

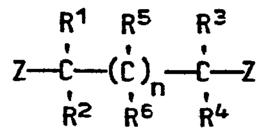 (III)

in welcher

$R^1$ bis $R^6$ und der Index n die oben angegebene Bedeutung haben und

Z für eine elektronenanziehende Abgangsgruppierung steht,

in Gegenwart einer starken Base und in Gegenwart eines aprotischen, polaren Verdünnungsmittels, oder in einem wäßrig-organischen Zweiphasensystem in Gegenwart eines Phasentransferkatalysators umsetzt.

An die so erhaltenen Verbindungen der Formel (I) kann gegebenenfalls anschließend eine Säure oder ein Metallsalz addiert werden.

Weiterhin wurde gefunden, daß die neuen cyclischen Azolylvinylether der Formel (I) starke fungizide Eigenschaften aufweisen. Ueberraschenderweise zeigen dabei die erfindungsgemäßen Verbindungen bessere fungizide Wirkungen als die aus dem Stand der Technik bekannten Verbindungen 2-(4-Chlorphenoxy)-4,4-dimethyl-1-(imidazol-1-yl)-1-penten-3-on und Zinkethylen-1,2-bisdithiocarbamidat. Die erfindungsgemäßen Wirkstoffe stellen somit eine Bereicherung der Technik dar.

Die erfindungsgemäßen cyclischen Azolylvinylether sind durch die Formel (I) allgemein definiert. In dieser Formel stehen vorzugsweise

A für ein Stickstoffatom oder die CH-Gruppe,

Ar für gegebenenfalls einfach oder mehrfach, gleich oder verschieden substituiertes Phenyl, wobei als Substituenten genannt seien: Halogen, Alkyl und Alkoxy mit jeweils 1 bis 4 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, Nitro, Cyano, jeweils gegebenenfalls durch Halogen substituiertes Phenyl und Phenoxy, sowie Cycloalkyl mit 5 oder 6 Kohlenstoffatomen;

$R^1$ bis $R^6$, die gleich oder verschieden sein können, für Wasserstoff oder geradkettiges oder verzweigtes Alkyl mit 1 bis 4 Kohlenstoffatomen, und der Index

n für 0 oder 1.

Besonders bevorzugt sind diejenigen Verbindungen der Formel (I), in denen

A für ein Stickstoffatom oder die CH-Gruppe steht,

Ar für gegebenenfalls einfach bis dreifach, gleich oder verschieden substituiertes Phenyl steht, wobei als Substituenten genannt seien: Fluor, Chlor, Brom, Methyl, Ethyl, Methoxy, Trifluormethyl, Trifluormethoxy, Trifluormethylthio, Nitro, Cyano, jeweils gegebenenfalls durch Chlor

oder Fluor substituiertes Phenyl oder Phenoxy, sowie Cyclohexyl,

$R^1$ bis $R^6$, die gleich oder verschieden sein können, für Wasserstoff, Methyl, Ethyl oder Isopropyl stehen; und der Index

n für 0 oder 1 steht.

Bevorzugte erfindungsgemäße Verbindungen sind auch Additionsprodukte aus Säuren und denjenigen cyclischen Azolylvinylethern der Formel (I), in denen die Substituenten A, Ar, $R^1$ bis $R^6$ und der Index n die Bedeutungen haben, die bereits vorzugsweise für diese Substituenten und den Index genannt wurden.

Zu den Säuren die addiert werden können, gehören vorzugsweise Halogenwasserstoffsäuren, wie z.B. Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salicylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen cyclischen Azolylvinylethern der Formel (I), in denen die Substituenten A, Ar, $R^1$ bis $R^6$ und des Index n die Bedeutungen haben, die bereits vorzugsweise für diese Substituenten und den Index genannt wurden.

Hierbei sind Salze des Kupfers, Zinks, Mangans, Magnesiums, Zinn, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Verwendet man beispielsweise 4-Biphenylyl-(1,2,4-triazol-1-yl-methyl)-keton und 1,3-Dibrompropan in Gegenwart von Natriumhydrid als Ausgangsstoffe, so kann der Reaktionsablauf des erfindungsgemäßen Verfahrens durch das folgende Formelschema wiedergegeben werden:

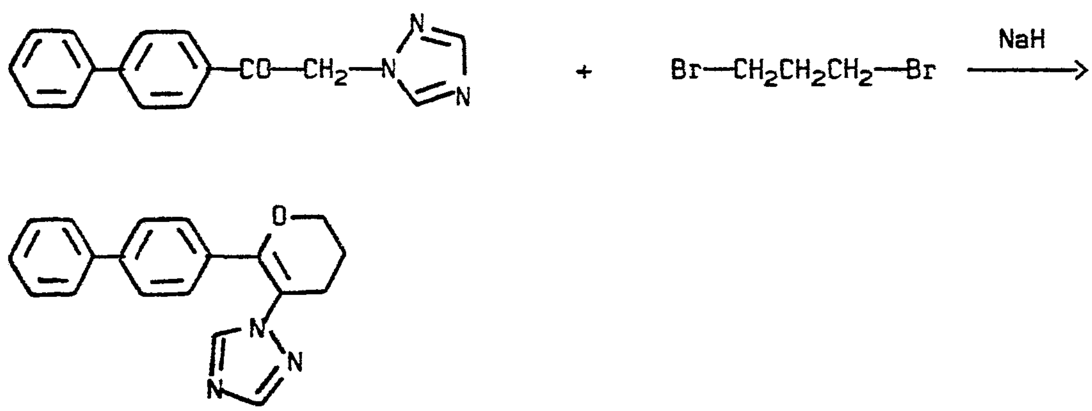


Die bei der Durchführung des erfindungsgemäßen Verfahrens als Ausgangsstoffe zu verwendenden Azolylmethyl-Ketone sind durch die Formel (II) allgemein definiert. In dieser Formel stehen A und Ar vorzugsweise für die Bedeutungen, die bereits im Zusammenhang mit der Beschreibung der erfindungsgemäßen Stoffe der Formel (I) vorzugsweise für diese Substituenten genannt wurden.

Die Azolylmethyl-Ketone der Formel(II) sind bekannt (vergleiche hierzu z.B. DE-OS 2 431 407, DE-OS 26 10 022 und DE-OS 26 38 470) und werden in allgemein bekannter Art und Weise erhalten, indem man die entsprechenden Halogenmethyl-Ketone mit Imidazol oder 1,2,4-Triazol in Gegenwart eines Verdünnungsmittels, wie beispielsweise Acetonitril, und in Gegenwart eines Säurebindemittels, wie beispielsweise Triethylamin, umsetzt.

Die außerdem für das erfindungsgemäße Verfahren als Ausgangsstoffe zu verwendenden Verbindungen sind durch die Formel (III) allgemein definiert. In dieser Formel stehen $R^1$ bis $R^6$ und der Index n vorzugsweise für diejenigen Bedeutungen, die bereits im Zusammenhang mit der Beschreibung der erfindungsgemäßen Stoffe der Formel (I) vorzugsweise für diese Substituenten und den Index genannt wurden. Z steht vorzugsweise für eine elektronenanziehende Abgangsgruppierung, wie beispielsweise Halogen, p-Methylphenylsulfonyloxy, die Gruppierung $-O-SO_2-OR$ oder $-NR_3$ und andere, wobei R hierbei z.B. für Alkyl mit 1 bis 4 Kohlenstoffatomen steht.

Die Verbindungen der Formel (III) sind allgemein bekannte Verbindungen der organischen Chemie, bzw. können sie in bekannter Art und Weise erhalten werden.

Für das erfindungsgemäße Verfahren kommen als Verdünnungsmittel aprotische, polare Lösungsmittel infrage. Hierzu gehören vorzugsweise Dimethylformamid, Dimethylacetamid, Dimethylsulfoxid, N-Methylpyrrolidin, 2-Pyrrolidinon und Hexamethylphosphorsäuretriamid. In manchen Fällen kann es vorteilhaft sein, diese Solventien mit anderen üblichen, inerten organischen Lösungsmitteln zu mischen, wie beispielsweise aromatische Kohlenwasserstoffe.

Das erfindungsgemäße Verfahren wird in Gegenwart einer starken Base durchgeführt. Hierbei können alle üblichen organischen und insbesondere anorganischen Basen eingesetzt werden, wie vorzugsweise Alkalihydride, -hydroxide oder -carbonate, beispielsweise Natriumhydrid und Natriumhydroxid, tert. Amine, wie Triethylamin, Piperidin und Pyridin.

Die Reaktionstemperaturen können bei der Durchführung des erfindungsgemäßen Verfahrens in einem größeren Bereich variiert werden. Im allgemeinen arbeitet man zwischen -20°C und 100°C vorzugsweise zwischen 20°C und 100°C.

Bei der Durchführung des erfindungsgemäßen Verfahrens setzt man auf 1 Mol Azolylmethyl-Keton der Formel (II) vorzugsweise 1 bis 2 Mol an Verbindung der Formel (III) ein. Die Isolierung der Endprodukte der Formel (I) erfolgt in allgemein üblicher Art und Weise. Dabei werden auch eventuell auftretende Nebenprodukte, die durch Alkylierung an der $CH_2$-Gruppe entstehen, in üblicher Weise, wie beispielsweise säulenchromatographisch oder durch Umkristallisation abgetrennt.

Das erfindungsgemäße Verfahren kann auch in einem Zweiphasensystem , wie beispielsweise wäßrige Natron- oder Kalilauge/Toluol oder Methylenchlorid, gegebenenfalls unter Zusatz von 0,1 bis 1 Mol eine Phasentransferkatalysators, wie beispielsweise Ammonium- oder Phosphoniumverbindungen, beispielsweise seien Benzyldodecyl-dimethyl-ammoniumchlorid und Triethyl-benzyl-ammoniumchlorid genannt, durchgeführt

werden.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und gegebenenfalls durch Waschen mit einem organischen Lösungsmittel gereinigt werden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Botrytis-Arten, wie beispielsweise Botrytis cinerea;
Plasmopara-Arten, wie beispielsweise Plasmopara viticola;
Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustilago-Arten, wie beispielsweise Ustilago nuda oder Ustilago avenae
Septoria-Arten, wie beispielsweise Septoria nodorum;
Tilletia-Arten wie beispielsweise Tilletia caries;
Xanthomonas-Arten, wie beispielsweise Xanthomonas oryzae;
Pseudomonas-Arten, wie beispielsweise Pseudomonas lachrymans;
Pyricularia-Arten, wie beispielsweise Pyricularia oryzae;
Pellicularia-Arten, wie beispielsweise Pellicularia sasakii ,
Pyrenophora-Arten, wie beispielsweise Pyrenophora teres (Konidienform: Drechslera, Syn. Helminthosporium),
Cochliobolus-Arten, wie beispielsweise Cochliobolus sativus, (Konidienform Drechslera, Syn. Helminthosporium) und
Cercospora-Arten, wie beispielsweise Cercospora canescens.

Die gute Pflanzenverträglichkeit der Wirkstoff in den zur Bekämpfung von Pflanzenkrankheiten notwenidigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Venturia-Arten, wie gegen den Erreger des Apfelschorfs (Venturia inaequalis); von Getreidekrankheiten, wie Cochliobolus sativus, Leptosphaeria nodorum, Erysiphe graminis und Pyrenophora teres , sowie von Reiskrankheiten, wie Pyricularia oryzae und Pellicularia sasakii, eingesetzt werden. Außerdem zeigen die erfindungsgemäßen Stoffe ein breites und gutes fungizides in-vitro-Wirkungsspektrum.

In entsprechenden Aufwandmengen zeigen die erfindungsgemäßen Stoffe auch bakterizide und wachstumsregulatorische Eigenschaften.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alzarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

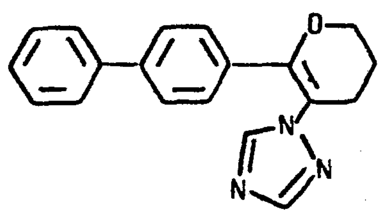

In eine Lösung von 26,4 g 1-(4-Phenylphenacyl)-1,2,4-triazol und 20,2 g 1,3-Dibrompropan in 100 ml trockenem Dimethylformamid gibt man bei Raumtemperatur im Verlauf von 4 Stunden 6 g Natriumhydrid (80%ig) zu. Man läßt 24 Stunden bei Raumtemperatur und 5 Stunden bei 30°C rühren und engt anschließend im Vakuum ein. Der Rückstand wird in 300 ml Essigester/Toluol (1:1) und 200 ml Wasser aufgenommen. Die organische Phase wird abgetrennt, zweimal mit Wasser gewaschen, über Natriumsulfat getrocknet und im Vakuum eingeengt. Der Rückstand wird mit 20 ml Ethanol und Wasser bis zur Trübung versetzt. Nach einigen Stunden wird der kristalline Niederschlag abgesaugt und aus 90%-igem Ethanol umkristallisiert.
Man erhält 12,2 g (40,3 % der Theorie) 1-(4-Biphenyl-yl)-2-(1,2,4-triazol-1-yl)-4,5-dihydropyran vom Schmelzpunkt 113°C.

In entsprechender Weise und gemäß den angegebenen Verfahrensbedingungen können die folgenden Verbindungen der Formel (I) erhalten werden:

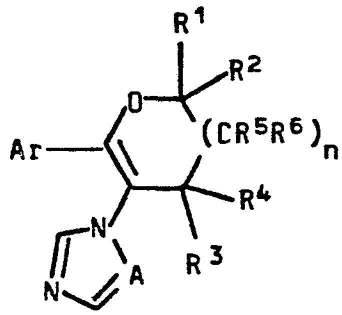

(I)

| Bsp. Nr. | A | Ar | $-CR^1R^2-(CR^5R^6)_n-CR^3R^4-$ | Smp. (°C) |
|---|---|---|---|---|
| 2 | N | 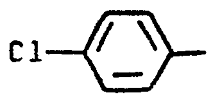 | $-CH_2-CH_2-CH_2-$ | 87-88 |
| 3 | CH | 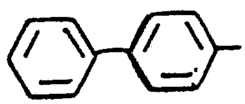 | $-CH_2-CH_2-CH_2-$ | 151 |

Verwendungsbeispiele

In den nachfolgenden Beispielen werden die nachstehend angegebenen Verbindungen als Vergleichssubstanzen eingesetzt:

(A) 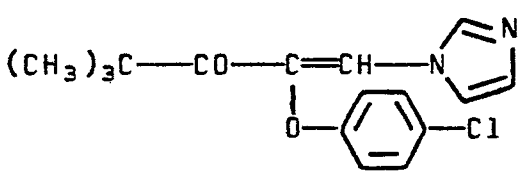

(B) 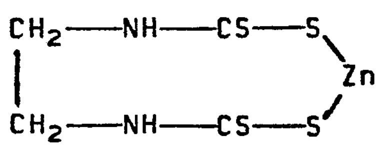

Beispiel A

Venturia-Test (Apfel) / protektiv

Lösungsmittel: 4 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelags werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Ueberlegenheit in der Wirksamkeit gegenüber dem Stand der Technik zeigen in diesem Test z.B. die Verbindungen gemäß folgender Herstellungsbeispiele: 1 und 3.

Beispiel B

Cochliobolus sativus-Test (Gerste) /protektiv

Lösungsmittel: ..100. Gewichtsteile .Dimethylformamid.......
Emulgator: .0,25. Gewichtsteile .Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß folgender Herstellungsbeispielen: 2 und 3

Beispiel C

Leptosphaeria nodorum-Test (Weizen) / protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.
Die Pflanzen werden im Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß folgender Herstellungsbeispiele: 1

## Patentansprüche

1. Cyclische Azolvinylether der allgemeinen Formel

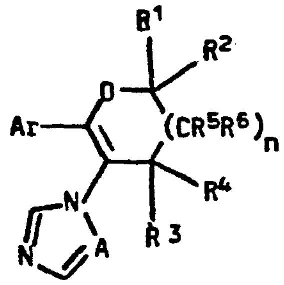

(I)

in welcher

A für ein Stickstoffatom oder die CH-Gruppe steht,

Ar für gegebenenfalls substituiertes Phenyl steht,

$R^1$ bis $R^6$ gleich oder verschieden sind und für Wasserstoff oder Alkyl stehen und

n für 0 oder 1 steht,

und deren Säureadditions-Salze und Metallsalz-Komplexe.

2. Cyclische Azolvinylether der Formel (I) gemäß Anspruch 1, worin

A für ein Stickstoffatom oder die CH-Gruppe steht,

Ar für gegebenenfalls einfach oder mehrfach, gleich oder verschieden durch Halogen, Alkyl und Alkoxy mit jeweils 1 bis 4 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, Nitro, Cyano, jeweils gegebenenfalls durch Halogen substituiertes Phenyl und Phenoxy, sowie Cycloalkyl mit 5 oder 6 Kohlenstoffatomen substituiertes Phenyl steht,

$R^1$ bis $R^6$, die gleich oder verschieden sein können, für Wasserstoff oder geradkettiges oder verzweigtes Alkyl mit 1 bis 4 Kohlenstoffatomen stehen, und der Index

n für 0 oder 1 steht.

3. Cyclische Azolvinylether der Formel (I) gemäß Anspruch 1, worin

A für ein Stickstoffatom oder die CH-Gruppe steht,

Ar für gegebenenfalls einfach bis dreifach, gleich oder verschieden durch Fluor, Chlor, Brom, Methyl, Ethyl, Methoxy, Trifluormethyl, Trifluormethoxy, Trifluormethylthio, Nitro, Cyano, jeweils gegebenenfalls durch Chlor oder Fluor substituiertes Phenyl oder Phenoxy, sowie durch Cyclohexyl substituiertes Phenyl steht,

$R^1$ bis $R^6$ die gleich oder verschieden sein können, für Wasserstoff, Methyl, Ethyl oder Isopropyl stehen, und der Index

n für 0 oder 1 steht.

4. Verfahren zur Herstellung von cyclischen Azolvinylethern der allgemeinen Formel

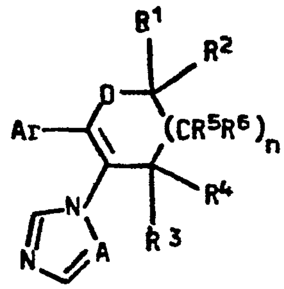

(I)

in welcher

A für ein Stickstoffatom oder die CH-Gruppe steht,

Ar für gegebenenfalls substituiertes Phenyl steht,

$R^1$bis$R^6$ gleich oder verschieden sind und für Wasserstoff oder Alkyl stehen und

n für 0 oder 1 steht,

und deren Säureadditions-Salze und Metallsalz-Komplexe, dadurch gekennzeichnet, daß man Azolylvinylketone der Formel

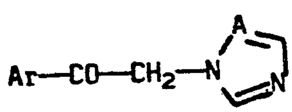

(II)

in welcher

A und Ar die oben angegebene Bedeutung haben,

mit Verbindungen der Formel (III)

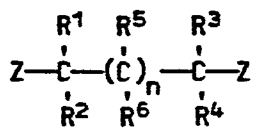 (III)

in welcher

$R^1$ bis $R^6$ und der Index n die oben angegebene Bedeutung haben und

Z für eine elektronenanziehende Abgangsgruppierung steht,

in Gegenwart einer starken Base und in Gegenwart eines aprotischen, polaren Verdünnungsmittels, oder in einem wäßrig-organischen Zweiphasensystem in Gegenwart eines Phasentransferkatalysators umsetzt und anschließend gegebenenfalls in Säureadditionssalze und Metallsalzkomplexe überführt.

5. Fungizide Mittel, gekennzeichnet durch einen Gehalt an mindestens einem cyclischen Azolvinylether der Formel (I).

6. Verfahren zur Bekämfpung von Pilzen, dadurch gekennzeichnet, daß man cyclische Azolvinylether der Formel (I) auf Pflanzen oder ihren Lebensraum einwirken läßt.

7. Verwendung von cyclischen Azolvinylethern der Formel (I) zur Bekämpfung von Pilzen.

8. Verfahren zur Herstellung von fungiziden Mitteln, dadurch gekennzeichnet, daß man cyclische Azolvinylether der Formel (I) mit Streckmitteln und/oder oberflächenaktiven Mitteln vermischt.